Europäisches Patentamt

European Patent Office

Office européen des brevets

(11) Numéro de publication: **0 490 768 B1**

# FASCICULE DE BREVET EUROPEEN

(45) Date de publication de fascicule du brevet: **05.04.95**

(51) Int. Cl.6: **A61K 9/20**, A61K 47/26, A23G 3/30, A23G 3/00

(21) Numéro de dépôt: **91403372.5**

(22) Date de dépôt: **12.12.91**

(54) **Composition pulvérulente directement compressible, et procédé d'obtention.**

(30) Priorité: **14.12.90 FR 9015708**

(43) Date de publication de la demande:
**17.06.92 Bulletin 92/25**

(45) Mention de la délivrance du brevet:
**05.04.95 Bulletin 95/14**

(84) Etats contractants désignés:
**AT BE CH DE DK ES FR GB GR IT LI LU NL SE**

(56) Documents cités:
**EP-A- 0 220 103**
**EP-A- 0 329 977**
**FR-A- 2 336 123**
**US-A- 3 899 593**
**US-A- 4 284 650**

**JAPANESE PATENTS GAZETTE Section Ch,**
**Week 7421, 1974 Derwent Publications**
**Ltd.,London, GB; Class B, AN 74-39213V**

(73) Titulaire: **Roquette Frères**

**F-62136 Lestrem (FR)**

(72) Inventeur: **Serpelloni, Michel**
**155 Route Nationale**
**F-62660 Beuvry Les Bethune (FR)**
Inventeur: **Croisier,Alain**
**20 Rue du Hallage**
**F-62400 Locon (FR)**

(74) Mandataire: **Boulinguiez, Didier et al**
**Cabinet Plasseraud**
**84, rue d'Amsterdam**
**F-75009 Paris (FR)**

**Description**

La présente invention concerne, à titre de produit industriel nouveau, une composition pulvérulente directement compressible à base de xylitol.

Elle a également pour objet un procédé de préparation de cette composition pulvérulente.

Elle concerne en outre l'utilisation de cette composition pulvérulente comme agent de charge édulcorante dans les comprimés et dans les articles de confiserie du type chewing-gum.

Le xylitol, sucre-alcool pentavalent, est un produit sucré cristallin, de couleur blanche, dénué d'odeur et soluble dans l'eau. Sous forme cristalline, il possède une chaleur de dissolution négative et provoque ainsi en bouche un effet rafraichissant, ou "cooling-effect", agréable. Cette propriété est particulièrement avantageuse pour des comprimés pharmaceutiques ou alimentaires.

Outre son "cooling-effect" le xylitol présente des qualités édulcorantes intéressantes. En effet, si l'on prend pour référence le saccharose, en lui attribuant une valeur édulcorante de 1, on constate que pour le xylitol, cette valeur édulcorante est du même ordre, tandis que pour d'autres polyols plus traditionnels tels que le sorbitol ou le mannitol, elle est respectivement de 0,60 et 0,45.

A l'instar des autres sucres-alcools déjà utilisés dans l'industrie alimentaire et en pharmacie, le xylitol est un substitut de saccharose avantageux pour les diabétiques.

En outre, le xylitol présente une propriété intéressante au regard de la santé dentaire qui le distingue des autres polyols connus. Il est en effet non seulement acariogène, c'est-à-dire qu'il ne peut servir de substrat aux bactéries présentes dans la cavité buccale, mais a également un rôle dans la prévention de la carie dentaire.

C'est donc à plusieurs titres que se justifierait l'emploi du xylitol comme agent liant et diluant dans le domaine de la fabrication de comprimés.

Il est bien connu que, par nature, certaines poudres se prêtent mal à la compression. C'est malheureusement le cas du xylitol poudre cristallisé qui ne permet pas d'obtenir des comprimés de dureté acceptable. Sa limite de compressibilité est relativement basse, ce qui favorise l'apparition du phénomène de "capping", c'est-à-dire un laminage du comprimé dans un plan diamétral qui lui supprime toute sa cohésion. Un tel comprimé est très fragile et a tendance à se rompre en deux ou plusieurs parties lors d'un test de dureté, voire dès son éjection de la presse.

L'une des techniques connues pour conférer aux poudres des aptitudes à la compression est la granulation humide. Il s'agit d'un procédé onéreux et relativement complexe à mettre en oeuvre, dans lequel on a recours à des additifs supplémentaires que sont les liants comme l'amidon, la cellulose ou la gélatine. En ce qui concerne le xylitol, il est en outre bien connu que ce produit se prête très mal à la granulation humide aqueuse, en raison de son hygroscopicité et de sa très forte solubilité dans l'eau. Il est donc nécessaire d'employer des solvants non aqueux tels que le polyéthylèneglycol ou l'éthanol, qui ne présentent pas cependant les mêmes avantages que l'eau sur le plan économique et de la toxicité. D'autre part, la compressibilité des granulés de xylitol ainsi préparés n'est pas bonne - cf Acta Pharmaceutica, Fennica 87, pages 61 à 73 (1978) et 91, page 48 (1982) -.

La technique dite de compression directe sans traitement préalable de la poudre reste donc la plus avantageuse, notamment sur le plan économique, pour l'industrie pharmaceutique et pour l'industrie alimentaire.

Pour tenter de rendre le xylitol directement compressible, il a tout d'abord été imaginé de mélanger le xylitol pulvérulent cristallisé avec différents liants connus comme par exemple la cellulose micro-cristalline - cf Acta Pharmaceutica Fennica 91 pages 47-54 (1982) -. Hormis le fait que ce composé soit insoluble, ce qui le rend difficilement applicable pour des comprimés à sucer - les plus courants en alimentaire -, il faut noter que la cellulose micro-cristalline est présente à raison de 10 à 50 % en poids par rapport au xylitol. Par conséquent, elle a tendance à masquer l'expression de certaines des propriétés avantageuses du xylitol cristallisé, telles que le pouvoir édulcorant, l'effet rafraichissant, la saveur agréable. Il est clair que cela diminue l'intérêt de l'emploi du xylitol cristallisé en compression. En outre, l'incidence économique de l'incorporation de cellulose micro-cristalline n'est pas à négliger, cet additif étant en effet relativement onéreux. Enfin, la présence de liant n'est pas très prisée par les fabricants de produits comprimés notamment pharmaceutiques.

La demande internationale de brevet WO 92/10168 décrit également un procédé de fabrication de granulés de xylitol directement compressible consistant en la granulation humide de xylitol finement broyé par emploi d'un liant pouvant être une solution de polydextrose, une solution de carboxyméthyl cellulose sodique ou encore un sirop de maltose hydrogéné. Un séchage final est obligatoire. On obtient ainsi des granules contenant de 0,1 % à 5 % de liant.

Une autre technique connue pour conférer aux poudres des aptitudes à la compression consiste à procéder à une extrusion. Cette méthode est notamment décrite pour le maltitol dans le brevet européen EP 220 103 et pour le xylitol dans le document JP 49-16929. Dans les deux cas, aucun additif n'est ajouté au polyol de sorte qu'il ne s'agit jamais d'une co-extrusion mais seulement d'une simple extrusion.

Il a été également proposé, dans la demande de brevet français n° 2.336.123, de fabriquer des tablettes à mâcher à partir de mélange à sec de xylitol en quantité de 10 à 80 % en poids et d'un polyol en quantité de 10 à 80 % en poids, par rapport au poids de la tablette. Le polyol peut être le sorbitol, le mannitol ou un mélange de ceux-ci. Il ressort de la description de cette demande de brevet que le polyol représente au moins 50 % en poids par rapport au xylitol. Ce dernier ne peut donc être considéré comme le constituant principal de la poudre à comprimer. Dans ces conditions, il n'est pas possible de bénéficier de manière optimale de tous les avantages propres au xylitol.

La demande de brevet européen n° 305 356 décrit un procédé de préparation de produits granulés utilisables en compression directe. Suivant ce procédé, un produit pulvérulent, qui peut être du xylitol, est mis en contact sous agitation avec un liquide constitué par le même produit fondu, le mélange obtenu étant ensuite rapidement refroidi. Compte tenu de cette dernière disposition, le produit obtenu présente une proportion non négligeable de forme amorphe. Cela n'est pas sans induire certains inconvénients comme notamment une perte de cooling-effect, une hygroscopicité plus élevée, donc la tendance au mottage de la poudre, et une faible comprimabilité. On notera également que ce procédé est relativement délicat à mettre en oeuvre. De plus, il a pu être vérifié que le produit granulé obtenu présente de mauvaises caractéristiques de friabilité et qu'il ne permet pas de préparer des comprimés ayant des propriétés de compression, à savoir notamment de dureté, notablement améliorées par rapport à celles de comprimés obtenus à l'aide de xylitol cristallisé traditionnel.

En agglomérant du xylitol poudre cristallisé de très fine granulométrie à l'aide d'un sirop de sorbitol sous forte agitation, la titulaire de la demande de brevet européen n° 329 977 propose un agent liant et diluant, utilisable en compression directe, dont les granulés ont une taille comprise entre environ 0,1 et 1 mm et contenant 94 à 98 % en poids de xylitol, 1 à 5 % en poids de sorbitol, 0 à 2 % en poids d'autres polyols et moins de 1 % en poids d'eau. Cet agent a une densité apparente ("tapée") de 0,7 à 0,8 g/cm$^3$.

Le broyage préalable du xylitol pour obtenir une fine granulométrie, l'agglomération à l'aide d'un sirop de sorbitol à faible matière sèche, ainsi que le séchage final de la poudre ne donnent pas au procédé toute la simplicité souhaitable et constituent des opérations supplémentaires qui augmentent son coût de mise en oeuvre.

Par ailleurs, on a pu constater que les résultats obtenus en termes de dureté pour les comprimés préparés à partir de cet agent liant et diluant ne sont pas satisfaisants.

C'est sur la base de ces dernières constatations et au vu de tous les inconvénients propres aux autres procédés de l'art antérieur que la demanderesse a cherché à mettre au point une composition pulvérulente pour compression directe à base de xylitol, c'est-à-dire principalement constituée par ce polyol, présentant des propriétés de comprimabilité et d'écoulement améliorées par rapport aux produits pulvérulents existants comprenant du xylitol.

Et c'est à la suite de nombreuses études et essais qu'elle a réussi à préparer une composition pulvérulente directement compressible à base de xylitol, caractérisée en ce qu'elle comprend au moins un additif choisi parmi les saccharides, oligosaccharides et polysaccharides et leurs correspondants hydrogénés, en ce qu'elle présente une comprimabilité, déterminée dans un test A, supérieure à 70N, de préférence supérieure à 80N, à l'exception des compositions (décrites dans la demande internationale de brevet WO 92/10168) préparées par granulation de xylitol avec un liant non-cariogène physiologiquement acceptable, ledit liant étant présent à raison de 0,1 % à 5 % en poids et étant sélectionné dans le groupe constitué par les sucres réducteurs polymérisés, les sels alcalins de carboxyméthylcellulose et les hydrolysats d'amidon hydrogénés.

De façon surprenante et inattendue, cette valeur de comprimabilité est de loin supérieure aux valeurs obtenues avec les compositions à base de xylitol connues jusqu'alors.

La proportion en xylitol de la composition est de préférence supérieure ou égale à 60 % en poids et plus préférentiellement encore à 80 % en poids.

Le test A consiste à mesurer la force, exprimée en Newton, qui est représentative de la comprimabilité de la composition étudiée et qui est nécessaire pour provoquer l'écrasement d'un comprimé préparé à partir de ladite composition, c'est-à-dire pour provoquer l'apparition de lignes de rupture au sein de la masse constitutive de celui-ci, cette force traduisant donc la résistance à l'écrasement du comprimé qui est cylindrique, à faces planes, d'un diamètre de 13 mm, d'une épaisseur de 4 mm et d'un poids de 0,717 g, c'est-à-dire d'une masse volumique apparente ou densité de 1,35 g/ml, ladite force étant exercée contre la surface périphérique du comprimé en direction de l'axe de révolution de celui-ci au moyen d'une butée

EP 0 490 768 B1

mobile s'appliquant contre ladite surface le long d'une génératrice, ledit comprimé étant par ailleurs immobilisé contre une butée fixe appliquée également contre la surface périphérique du comprimé le long d'une génératrice diamétralement opposée à celle contre laquelle s'applique la butée mobile.

Pour préparer ces comprimés, on ajoute à la composition étudiée 2 % en poids de lubrifiant, à savoir du stéarate de magnésium.

Ces deux produits sont homogénéisés l'un à l'autre à l'aide d'un mélangeur TURBULA T2C (commercialisé par la Firme WILLY A. BACHOFEN AG, Suisse) pendant cinq minutes à la vitesse d'entraînement de 42 tours/minute.

Pour effectuer la compression du mélange obtenu, on a recours à une presse alternative FROGERAIS de type AM. Cette presse est équipée de poinçons ronds à faces planes de diamètre égal à 13 millimètres.

Pour parvenir aux caractéristiques des comprimés mentionnés ci-dessus, on règle sur la presse l'enfoncement du poinçon supérieur et le volume de remplissage de la matrice, cette dernière disposition permettant de fixer la quantité en poids de mélange pulvérulent souhaitée, en l'espèce 0,717 g.

Pour l'évaluation de la résistance à l'écrasement de ces comprimés, on utilise un duromètre SCHLEUNIGER 2E (commercialisé en France par les Etablissements FROGERAIS).

La composition suivant l'invention comprend au moins un additif choisi parmi les saccharides, oligosaccharides et polysaccharides et leurs correspondants hydrogénés, c'est-à-dire parmi les molécules organiques caractérisées par la présence de chaînons carbonés porteurs de groupements hydroxyles et de fonctions aldéhydiques, cétoniques ou acides.

Il s'agit de préférence des sucres tels que les aldoses et les cétoses ainsi que leurs dérivés hydrogénés, comme par exemple le sorbitol, le mannitol ou le maltitol ou les polysaccharides dont notamment les polymères de glucose, comme les maltodextrines ayant un Dextrose Equivalent inférieur à 20, ou les sirops de glucose, ainsi que leurs dérivés hydrogénés.

Conformément à une disposition avantageuse de l'invention, l'additif est choisi parmi la liste non limitative de composés suivants : sorbitol, maltitol, mannitol, maltodextrine. Dans un mode préféré de mise en oeuvre de l'invention, l'additif sélectionné est le sorbitol.

Il va de soi que l'invention ne se limite pas à l'emploi d'un seul additif en complément du xylitol, mais englobe aussi toute composition comprenant plusieurs additifs de nature différente.

La présente invention concerne également un procédé de préparation de la susdite composition pulvérulente à base de xylitol.

Conformément à ce procédé, on soumet une matière première essentiellement constituée de xylitol, c'est-à-dire dans une quantité supérieure ou égale à 60 %, et de préférence à 80 %, et d'au moins un additif, à un traitement d'extrusion à l'intérieur d'une installation d'extrusion comprenant une zone de traitement thermique et au moins une filière d'extrusion, le débit d'alimentation de l'installation en xylitol et en additif ainsi que les paramètres du traitement d'extrusion, à savoir la température régnant à l'intérieur de la zone de traitement thermique, le diamètre de la filière d'extrusion et la vitesse d'entraînement de la matière première à l'intérieur de la zone de traitement thermique étant sélectionnés de façon telle qu'à la sortie de la filière et avant la sortie de cette dernière, le mélange xylitol/additif soit partiellement fondu.

La composition pulvérulente à base de xylitol conforme à l'invention est également définie comme étant une composition pulvérulente susceptible d'être obtenue par le procédé conforme à l'invention.

De préférence, la susdite installation est du type bi-vis comportant au moins une filière d'extrusion, les paramètres du traitement d'extrusion étant sélectionnés de façon telle que la matière première se trouve à une température comprise entre 75 et 110°C à l'intérieur de la filière et avant la sortie de ladite matière première de cette dernière, ladite température étant dépendante de la nature et de la quantité de (ou des) l'additif(s) mis en oeuvre.

Cette température peut être aisément déterminée par l'homme du métier, sachant que la température de fusion du xylitol est d'environ 92°C et que la proportion de matière première fondue doit de préférence être comprise entre 30 et 90 %, et plus préférentiellement encore entre 50 et 80 %, de manière à obtenir en sortie d'extrusion un produit d'une viscosité telle que soient rendues aisées et rapides les opérations ultérieures du procédé.

Dans le cas où la composition pulvérulente comprend un seul additif et où ce dernier est du sorbitol, la température de la matière première à l'intérieur de la filière et avant la sortie de cette dernière, est, de préférence, comprise entre 80 et 105°C.

Dès lors que l'additif unique est constitué de maltitol, ladite température est de préférence comprise entre 80 et 100°C.

Avantageusement, le xylitol et l'additif constituant la matière première destinée à être soumise au traitement d'extrusion se présentent sous forme pulvérulente cristallisée ou non.

4

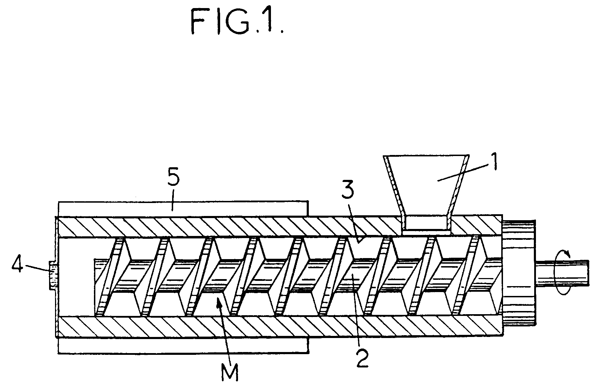

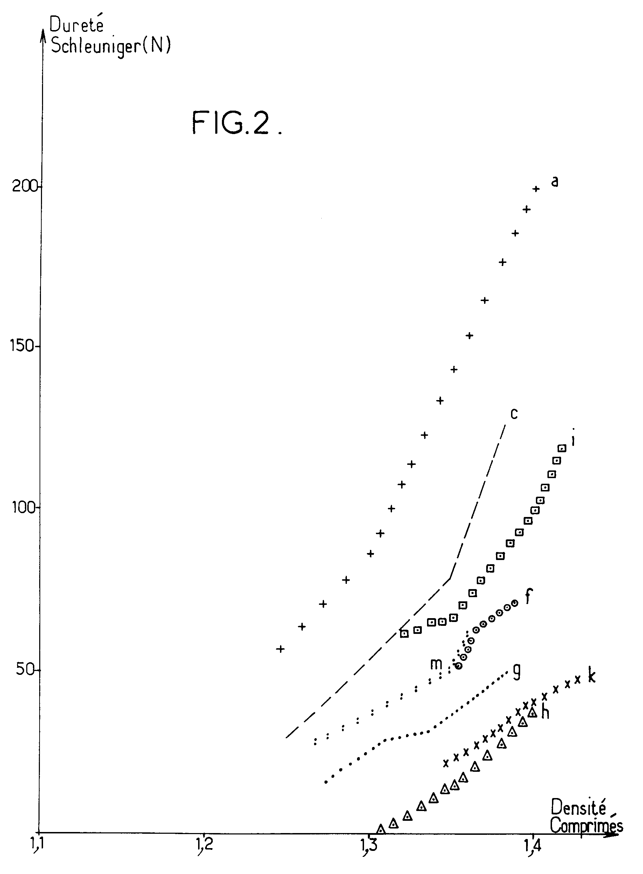

L'invention vise encore d'autres dispositions qui s'utilisent de préférence en même temps et dont il sera plus explicitement question ci-après et elle pourra, de toute façon, être bien comprise à l'aide du complément de description qui suit ainsi que des figures 1 et 2 annexées et des exemples.

La figure 1 représente, en coupe schématique, une installation d'extrusion du type de celles pouvant être utilisées dans le cadre du procédé selon l'invention.

Se proposant, par conséquent, de fabriquer une composition pulvérulente directement compressible à base de xylitol conforme à l'invention, on s'y prend comme suit ou de façon équivalente.

La matière première qui est soumise au traitement d'extrusion selon le procédé conforme à l'invention, est constituée, par exemple, d'un mélange de xylitol poudre à raison de 90 % en poids et de sorbitol poudre à raison de 10 % en poids.

Dans la pratique, on utilise du xylitol et du sorbitol cristallisés obtenus selon des techniques communément mises en oeuvre dans le domaine considéré.

L'installation d'extrusion est constituée avantageusement par une extrudeuse du type bi-vis comprenant, comme montré à la figure 1 :

- un système d'alimentation, notamment une trémie doseuse 1,
- un système de malaxage M, comprenant un système à deux vis sans fin 2 disposées à l'intérieur d'un carter 3, notamment en acier nitruré, et entraînées en rotation par un mécanisme non montré,
- une sortie comprenant une ou plusieurs filières 4 de différentes formes,
- des moyens de régulation thermique 5 permettant de contrôler la température de la zone de malaxage, ces moyens de régulation thermique 5 étant constitués, d'une part, de moyens de chauffage formés par exemple par des résistances électriques ou par un système de chauffage par induction ou à la vapeur, et d'autre part, de moyens de refroidissement non montrés disposés à l'extérieur du carter ou à l'intérieur et se présentant par exemple sous la forme de serpentins logés dans le carter, et/ou d'un circuit de fluide de réfrigérant logé à l'intérieur de la vis.

La matière première entrant par le système d'alimentation dans la zone de malaxage est soumise, grâce à la compression réalisée dans les spires de la vis, à un cisaillement et à un frottement mécanique intenses simultanément à l'échauffement induit par les moyens de chauffage qui sont mis en oeuvre.

L'extrusion constitue, par conséquent, un traitement thermo-mécanique.

Pour fixer les idées, on signale qu'on a obtenu de bons résultats avec une extrudeuse du type bi-vis mise sur le marché sous la dénomination "BC 82" par la Société CLEXTRAL. Les deux vis se copénètrent et tournent dans le même sens. La zone de malaxage est chauffée par induction ou refroidie par circulation d'un fluide réfrigérant dans un serpentin, et la température peut donc y être facilement régulée.

L'avantage essentiel de ce mode de chauffage est sa souplesse d'utilisation et son contrôle aisé au moyen d'une boucle de régulation simple (thermocouple/dispositif de commande des moyens de chauffage par induction ou des moyens de refroidissement).

L'installation utilisée dans le cadre de cet exemple comporte quatre filières de forme cylindrique et de diamètre de 5 mm.

La température de la zone de traitement thermique est obtenue en imposant au système de régulation une valeur prédéterminée. Dans le cas de l'installation d'extrusion dont il vient d'être question, cette valeur est comprise entre 75 et 110°C, de préférence entre 80 et 105°C.

Les caractéristiques mécaniques des vis et leur vitesse de rotation sont choisies de façon telle que la durée du séjour de la matière première à l'intérieur de la zone de traitement thermique soit de 5 à 300 s.

Grâce au choix de l'ensemble de ces paramètres, la température de la matière première ayant subi le traitement est de 75 à 110°C à l'intérieur des filières et avant sa sortie de ces dernières.

Le mélange coextrudé à base de xylitol obtenu à la sortie de l'installation d'extrusion est alors successivement soumis :

- à un refroidissement,
- à un broyage,
- et à un tamisage.

Suivant un autre aspect de la présente invention, la composition pulvérulente conforme à l'invention s'est révélée être particulièrement adaptée comme édulcorant dans les confiseries et en particulier dans les confiseries du type chewing-gum.

L'un des problèmes classiques auquel sont confrontés les fabricants lors de la préparation industrielle de chewing-gums, est le défaut de "machinabilité" de la masse de gomme base et de charge édulcorante travaillée. La manipulation de cette masse s'opère traditionnellement à une température de 40 - 50°C. Il est important qu'à cette température, la masse soit suffisamment molle pour permettre un mélange homogène de tous les constituants du chewing-gum (gomme base, charge édulcorante, arômes, colorants...), sans toutefois l'être trop, de façon à éviter les problèmes de collage dans les étapes de malaxage, de formage et de découpe.

La charge édulcorante influe, de manière notable, sur la viscosité de la masse travaillée. On sait, par exemple, lorsqu'il s'agit de poudre, qu'une très fine granulométrie permet d'obtenir une bonne dureté à chaud. L'inconvénient de ces poudres fines est leur très forte hygroscopicité, qui se traduit par une tendance au mottage, lors du stockage.

De façon surprenante et inattendue, la composition suivant l'invention permet d'obtenir des chewing-gums présentant une très bonne dureté à chaud et à froid, sans qu'il soit nécessaire de recourir à de très fines granulométries. Par ailleurs, la qualité organoleptique de ces chewing-gums est tout à fait satisfaisante.

La présente invention concerne donc également l'utilisation de la composition pulvérulente à base de xylitol conforme à l'invention à titre d'agent de charge édulcorante dans des confiseries du type chewing-gum et concerne également les confiseries ainsi obtenues.

La forte saveur sucrée, le cooling effect et l'effet cariostatique du xylitol sont des propriétés particulièrement appréciées dans l'application confiserie du type chewing-gum.

Les exemples donnés ci-après illustrent les différences significatives existant entre les compositions pulvérulentes conformes à l'invention et les produits à base de xylitol pour compression directe soit de l'art antérieur, soit élaborés à des fins comparatives par la Société Demanderesse, ainsi que les avantages procurés par l'utilisation desdites compositions pulvérulentes dans les confiseries du type chewing-gum.

**EXEMPLES RELATIFS A L'UTILISATION EN COMPRESSION**

Dans ces exemples, on utilise :
- cinq échantillons différents de la composition pulvérulente suivant l'invention, désignés par les références a, b, c, d, e;
- un échantillon de XYLITOL DC commercialisé par la Société CULTOR et désigné par la référence f;
- un échantillon de xylitol poudre extrudé présentant une pureté chimique minimale de 99 % en poids sur sec et désigné par la référence g;
- un échantillon de xylitol poudre commercialisé par la Société ROQUETTE et présentant une pureté chimique minimale de 99 % en poids sur sec et désigné par la référence h;
- trois échantillons de mélanges pulvérulents comprenant du xylitol poudre et du sorbitol poudre commercialisés par la Société ROQUETTE dont les puretés chimiques minimales sont respectivement de 99 % et 96 % en poids sur sec et mélangés l'un à l'autre à sec; les proportions pondérales de ces mélanges xylitol/sorbitol sont respectivement de 50/50 pour l'échantillon désigné par la référence i, de 90/10 pour l'échantillon désigné par la référence k et de 95/5 pour l'échantillon désigné par la référence l;
- un échantillon j de xylitol compressible préparé selon le procédé décrit dans la demande de brevet EP 0 305 356, par mélange de 300 g de xylitol fondu à une température d'environ 100 °C et de 700 g de xylitol cristallisé;
- et deux échantillons de mélanges pulvérulents constitués de xylitol et de sorbitol extrudés séparément puis mélangés l'un avec l'autre à sec; les puretés chimiques minimales du xylitol et du sorbitol sont respectivement de 99 % et 96 % en poids sur sec; les proportions pondérales de ces mélanges xylitol/sorbitol sont respectivement de 90/10 pour l'échantillon désigné par la référence m, et de 98/2 pour l'échantillon désigné par la référence n.

Les cinq échantillons f à j appartiennent à l'art antérieur, tandis que les quatre échantillons k à n ont été sélectionnés et élaborés par la Demanderesse à des fins comparatives.

1. Préparation des cinq échantillons a-e conformes à l'invention

Pour les trois échantillons a-c, l'additif choisi est du sorbitol du type de celui commercialisé par la demanderesse sous la marque déposée NEOSORB® P60. Les quantités de sorbitol sont respectivement de 10 %, 5 % et 2 % en poids par rapport à la composition pour les échantillons a, b et c. Pour l'échantillon d, l'additif choisi est le maltitol poudre, présent à raison de 10 % en poids par rapport à la composition.

Enfin, pour l'échantillon e, l'additif choisi et présent à raison de 10 % en poids par rapport à la composition est une maltodextrine issue de l'hydrolyse enzymatique d'amidon, ayant un Dextrose-Equivalent ou D.E. (nombre de grammes de sucres réducteurs exprimés en dextrose pour 100 grammes de produit sec) égal à 12, commercialisée par la demanderesse sous la marque déposée GLUCIDEX® 12.

Le xylitol employé dans chacun des échantillons a à e est obtenu par cristallisation dans l'eau et présente une richesse en xylitol minimale de 99 % en poids sur matières sèches.

Les échantillons a à e sont préparés par introduction dans l'extrudeuse d'un mélange pulvérulent homogène de xylitol et de l'additif dans les proportions citées ci-dessus. L'extrudeuse utilisée est celle dont il a été question plus haut, c'est-à-dire la "BC 82" de la Société CLEXTRAL.

La vitesse des vis est réglée de manière telle que le débit de l'installation soit de 200 kg/heure et que le temps de passage de la matière première dans l'installation soit de 30 s environ

La température de consigne du système de chauffage est programmée à une valeur comprise entre 85 et 110°C qui varie en fonction de la nature et de la quantité de l'additif.

Pour les échantillons a à e, les températures choisies sont les suivantes :

- échantillon a ≃ 90°C,
- échantillon b ≃ 92°C,
- échantillon c ≃ 93°C,
- échantillon d ≃ 90°C,
- échantillon e ≃ 92°C.

En sortie d'extrudeuse et après refroidissement, les mélanges coextrudés xylitol/additifs se présentent sous la forme de bâtonnets qui sont broyés à l'aide d'un broyeur du type à marteaux.

La fraction retenue est de granulométrie supérieure à 50 microns, plus généralement à 100 microns.

2. Tests

pour chacun des échantillons a à n testés, on a sélectionné une granulométrie moyenne comprise entre 400 et 900 microns.

En ayant recours à la méthode de CARR telle que décrite par CARR R.L. dans Chem. Eng. 72, n° 163, 168 (1985) et Chem. Eng. 72, n° 2, 69-73 (1985), on mesure l'indice d'écoulement et la densité des poudres a à n. L'appareil utilisé pour ce test est celui connu sous la marque HOSOKAWA POWDER TESTER et fabriquée par MICROMERITICS, Osaka (Japon).

On détermine, de plus, la friabilité de certaines des compositions pulvérulentes a à m. Cette propriété est caractérisée par le pourcentage de particules n'ayant pas résisté à un concassage dans un appareil appelé friabilimètre. En l'occurrence, on a utilisé celui de marque ERWEKA TA. Cet appareil contient cinq billes d'acier identiques de 1,7 cm de diamètre et de 18,87 g chacune. On y introduit 15 g d'une fraction granulométrique de 400 à 500 microns de la poudre testée et on met l'appareil en rotation à 25 tours minutes pendant quinze minutes. On détermine par pesée, à la fin du concassage, la proportion, exprimée en pourcentage, que représente le résidu retenu par un tamis de largeur de maille de 351 microns; la valeur de la friabilité correspond au complément à 100 g de cette dernière valeur. Plus le chiffre ainsi obtenu est grand, plus la friabilité est grande.

Enfin, on détermine par le test A défini plus haut la comprimabilité de ces échantillons.

Les résultats de ces mesures de comprimabilité selon le test A ainsi que la granulométrie moyenne, les indices d'écoulement et la friabilité des échantillons testés, sont réunis dans le tableau ci-après.

| Compositions pulvérulentes à base de xylitol | Echantillons a à e conformes à l'invention | | | | | Echantillons f à j de l'art antérieur | | | | | Echantillons comparatifs k à n | | | |
|---|---|---|---|---|---|---|---|---|---|---|---|---|---|---|
| | a | b | c | d | e | f | g | h | i | j | k | l | m | n |
| Richesse en xylitol | 90 | 95 | 98 | 90 | 90 | 98 | 100 | 100 | 50 | 100 | 90 | 95 | 90 | 98 |
| Granulométrie moyenne en microns | 700 | 750 | 810 | 820 | 840 | 410 | 675 | 700 | 650 | 480 | 680 | 690 | 700 | 810 |
| Densité poudre | 0,720 | 0,760 | 0,809 | 0,771 | 0,746 | | | | | 0,730 | | | | |
| Teneur en eau (%) | 0,40 | 0,30 | 0,25 | 0,30 | 0,60 | 0,30 | | | | | | | 0,30 | |
| Ecoulement (CARR) | SUPERIEUR À 75 | | | | | | | | | | | | | |
| Friabilité (%) | 63 | 71 | 82 | 78 | 80 | 93 | 40 | 15 | 65 | 65 | 25 | 25 | 53 | 48 |
| Comprimabilité (Résistance à la rupture) en N pour d = 1,35 | 144 | 130 | 83 | 89 | 102 | 49 | | | | | | | 80 | |

Il résulte de ce tableau que la composition pulvérulente conforme à l'invention présente une comprimabilité ou résistance à l'écrasement nettement accrue, toujours supérieure à 70 N. En comparaison, l'échantillon f constitué par le XYLITOL DC de la Société CULTOR et conforme à celui décrit et revendiqué dans la demande de brevet européen n° 0 329 977, possède une comprimabilité seulement égale à 49 N, c'est-à-dire trois fois moindre que celle de l'échantillon a conforme à l'invention (144 N). Il en va de même pour l'échantillon j correspondant au xylitol décrit dans la demande de brevet européen n° 0 305 356.

Ces différences extrêmement significatives montrent bien le caractère surprenant et inattendu des performances en compression de la composition pulvérulente suivant l'invention.

Il faut également remarquer que la comprimabilité d'une composition formée d'un mélange à sec 50/50 de xylitol et de sorbitol (échantillon i), c'est-à-dire répondant aux caractéristiques revendiquées dans la

8

demande de brevet français n° 2.336.123 citée ci-dessus ne s'élève qu'à une valeur de 65 N, nettement inférieure donc aux 83 N, voire aux 144 N, des compositions suivant l'invention. Cet inconvénient s'ajoute au fait qu'une proportion de 50 % de sorbitol est très importante et diminue d'autant la quantité de xylitol présent. Finalement, cela ne permet pas de profiter au mieux des vertus du xylitol à la consommation.

Le tableau montre également que la composition pulvérulente suivant l'invention possède avantageusement des caractéristiques de friabilité améliorées par rapport à celles des poudres de l'art antérieur et des poudres comparatives.

De façon à illustrer mieux encore l'amélioration des performances en compression directe des compositions suivant l'invention par rapport à celles des compositions de l'art antérieur, on a représenté sur la figure 2 en annexe, pour chacun des échantillons a, c, f, g, h, i, k, m, un graphe donnant l'évolution de la comprimabilité en N en fonction de la densité ou masse volumique apparente des comprimés en g/ml.

Les comprimés de densités différentes sont fabriqués de la même façon que celle décrite ci-dessus à l'aide d'une presse alternative AM. FROGERAIS. C'est en faisant varier la quantité en poids de poudre utilisée pour préparer un comprimé que l'on obtient différentes densités de comprimés. Plus cette quantité augmente, plus la densité du comprimé et la force de compression pour obtenir un comprimé aux dimensions données croissent.

On peut constater au vu de cette figure 2 que la comprimabilité des compositions a et c de l'invention est toujours nettement supérieure à celle des compositions f, g, h, i, j de l'art antérieur et des compositions comparatives k, m, quelle que soit la densité des comprimés.

Cette figure 2 montre notamment que les échantillons a et c de l'invention dont les proportions respectives en xylitol/sorbitol sont de 90/10 et 98/2, ont de meilleures aptitudes à la compression que le xylitol cristallisé extrudé (échantillon g) et que le xylitol cristallisé dans l'eau (échantillon h).

Si l'on compare les courbes caractéristiques des échantillons a et f, c'est-à-dire correspondant respectivement à la composition suivant l'invention xylitol/sorbitol coextrudés 90/10 et à la composition de XYLITOL DC de la Société CULTOR : Xylitol/Sorbitol 98/2 (sorbitol ajouté sous forme de sirop aqueux à la poudre de xylitol) conforme à la demande de brevet Ep 329 977, la nette supériorité en compression de la composition suivant l'invention est manifeste quelle que soit la densité du comprimé.

Rien ne pouvait laisser prévoir un tel gain au niveau des performances, sachant que l'on emploie dans les deux cas le sorbitol en association avec le xylitol.

En ce qui concerne les mélanges xylitol/sorbitol non extrudés dans les proportions 50/50 (échantillon i) conforme à la demande de brevet FR 2.336.123 et 90/10 (échantillon k comparatif), l'écart existant entre la courbe a et les courbes i et k montre une nouvelle fois le caractère très avantageux des compositions suivant l'invention. Même pour l'échantillon k équivalent sur le plan des proportions pondérales xylitol/sorbitol à l'échantillon a de l'invention, la supériorité en compression de ce dernier ressort clairement de l'observation des courbes a et k.

L'échantillon comparatif m formé de 90 % de xylitol, et de 10 % de sorbitol extrudés séparément donne une courbe m qui traduit la moindre comprimabilité de cet échantillon par rapport aux échantillons a à c de l'invention. Le simple mélange de xylitol et de sorbitol extrudés séparément ne permet pas d'améliorer la comprimabilité. Par conséquent, même si cette solution avait été décrite dans l'art antérieur, elle n'aurait certainement pas incité l'homme du métier à recourir à l'extrusion et encore moins à la coextrusion pour conférer une comprimabilité satisfaisante au xylitol. La démarche de la demanderesse pour parvenir à l'invention n'en est donc que plus méritoire.

## EXEMPLE RELATIF A L'UTILISATION DANS LES CONFISERIES DU TYPE CHEWING-GUM

Dans cet exemple, on a voulu comparer la dureté et les qualités organoleptiques de chewing-gums sans sucre préparés, d'une part à l'aide de l'échantillon g de xylitol pulvérulent connu (cristallisé dans l'eau), et d'autre part à l'aide de la composition pulvérulente a conforme à l'invention.

Les granulométries des deux poudres a et g sont identiques. Elles ne contiennent pas de particules de taille supérieure à 160 micromètres.

Composition des chewing-gums

```
- Gomme base ............................. 33 % en poids
  (gomme base 34/42 de la Société DREYFUS)


- Charge édulcorante ..................... 65 % en poids


- Lécithine .............................. 0,5 % en poids
  (Mc Thin AF1 de la Société LUCAS MEYER)


- Arôme menthe ........................... 1,5 % en poids
```

Mode opératoire

 . Préchauffer la gomme base vers 40 - 50°C et l'introduire dans le pétrin. Pétrir 2 minutes
 . Ajouter la moitié de la charge édulcorante pulvérulente et l'arôme et pétrir 3 minutes
 . Ajouter la lécithine et pétrir 1 minute
 . Ajouter le reste de la charge édulcorante, puis pétrir 4 minutes

Résultats

  On a mesuré par pénétrométrie, à l'aide d'un appareil du type INSTRON, les duretés des chewing-gums à différentes températures après un délai donné de stockage postérieurement à la fabrication, et après différentes durées de stockage à une température de 20°C.
  Les résultats sont donnés dans le tableau ci-après.

| Conditions de mesure | Chewing-gum préparé avec l'échantillon g | Chewing-gum préparé avec l'échantillon a conforme à l'invention |
|---|---|---|
| 45°C | 1,82 | 3,00 |
| 35°C | 6,57 | 8,82 |
| 24°C | 20,54 | 24,96 |
| après 1 j à 20°C | 23,65 | 26,60 |
| après 15 j à 20°C | 25,67 | 30,05 |

  Il ressort clairement de ce tableau que la composition a suivant l'invention confère au chewing-gum une dureté à chaud supérieure à celle obtenue avec l'échantillon g de l'art antérieur.
  Les chewing-gums comprenant l'échantillon a suivant l'invention peuvent donc être fabriqués beaucoup plus facilement. La machinabilité de la masse travaillée est nettement améliorée et les problèmes de collage aux équipements sont ainsi supprimés.
  S'agissant de la dureté à "froid" (20°C), les chewing-gums avec l'échantillon a ont également une dureté supérieure à celle des chewing-gums préparés avec l'échantillon g, tout en restant dans des limites acceptables.

On constate également que la dureté de ces deux types de chewing-gums évolue sensiblement de la même façon, au cours du stockage à cette température de 20°C.

En ce qui concerne l'aspect et la structure des chewing-gums obtenus, on observe que les chewing-gums préparés avec l'échantillon a ont l'avantage d'avoir une structure plus homogène, plus liée et d'être par ailleurs plus blancs que ceux préparés avec l'échantillon g de l'art antérieur.

Enfin, sur le plan organoleptique, les chewing-gums du type a et ceux du type g sont comparables. On note toutefois un effet sucrant se prolongeant plus longtemps pour le chewing-gum du type a.

Les avantages de l'emploi de la composition pulvérulente suivant l'invention ressortent donc clairement de l'exemple ci-dessus.

## Revendications

1. Composition pulvérulente directement compressible à base de xylitol, caractérisée en ce qu'elle comprend au moins un additif choisi parmi les saccharides, oligosaccharides et polysaccharides et leurs correspondants hydrogénés, en ce qu'elle présente une comprimabilité, déterminée dans un test A, supérieure à 70N, de préférence supérieure à 80N, à l'exception des compositions préparées par granulation de xylitol avec un liant non-cariogène physiologiquement acceptable, ledit liant étant présent à raison de 0,1 % à 5 % en poids et étant sélectionné dans le groupe constitué par les sucres réducteurs polymérisés, les sels alcalins de carboxyméthylcellulose et les hydrolysats d'amidon hydrogénés.

2. Composition selon la revendication 1, caractérisée en ce que la proportion en xylitol est supérieure ou égale à 60 % en poids et de préférence à 80 % en poids.

3. Composition selon la revendication 1 ou 2, caractérisée en ce que l'additif est choisi parmi les sucres, les sirops de glucose, les maltodextrines et leurs dérivés hydrogénés.

4. Composition selon la revendication 3, caractérisée en ce que l'additif est choisi dans le groupe comprenant le sorbitol, le mannitol, le maltitol, les maltodextrines.

5. Procédé de fabrication d'une composition pulvérulente directement compressible caractérisé en ce que l'on soumet une matière première essentiellement constituée de xylitol et d'au moins un additif choisi parmi les saccharides, oligosaccharides et polysaccharides et leurs correspondants hydrogénés, a un traitement d'extrusion comprenant une zone de traitement thermique et au moins une filière d'extrusion, le débit d'alimentation de l'installation en matière première ainsi que les paramètres de traitement d'extrusion, à savoir la température régnant à l'intérieur de la zone de traitement thermique, le diamètre de la filière d'extrusion et la vitesse d'entraînement de la matière première à l'intérieur de la zone de traitement thermique étant sélectionnés de façon telle qu'à la sortie de la filière et avant sa sortie de cette dernière, le mélange xylitol/additif soit partiellement fondu.

6. Procédé selon la revendication 5, caractérisé en ce que la matière première soumise à l'extrusion présente une quantité de xylitol supérieure ou égale à 60 % en poids, de préférence supérieure ou égale à 80 % en poids.

7. Procédé selon la revendication 5 ou la revendication 6 caractérisé en ce que l'additif est choisi parmi le sorbitol, le maltitol, le mannitol, les maltodextrines, l'additif préféré étant le sorbitol.

8. Procédé selon l'une quelconque des revendications 5 à 7, caractérisé en ce que l'installation est du type bivis comportant au moins une filière d'extrusion, les paramètres du traitement d'extrusion étant sélectionnés de façon telle que la matière première se trouve à une température comprise entre 75 et 110°C à l'intérieur de la filière et avant la sortie de ladite matière première de cette dernière.

9. Procédé selon l'une quelconque des revendications 5 à 8 dans lequel la composition pulvérulente comporte un additif constitué par du sorbitol, caractérisé en ce que la température de la matière première à l'intérieur de la filière et avant sa sortie de cette dernière, est, de préférence, comprise entre 80 et 105°C.

**10.** Composition pulvérulente directement compressible, caractérisée en ce qu'elle est susceptible d'être obtenue par le procédé selon l'une quelconque des revendications 5 à 9.

**11.** Utilisation de la composition pulvérulente selon l'une quelconque des revendications 1 à 4 et 10 à titre d'agent de charge édulcorante dans des comprimés ou dans des articles de confiserie, notamment du type chewing-gum.

**12.** Comprimés ou articles de confiserie, notamment du type chewing-gum, caractérisés en ce que leur charge édulcorante est constituée, au moins en partie, par la composition pulvérulente selon l'une quelconque des revendications 1 à 4 et 10.

**Claims**

**1.** Directly compressible pulverulent composition based on xylitol, characterized in that it comprises at least one additive selected from saccharides, oligosaccharides and polysaccharides and their corresponding hydrogenated compounds, in that it has a compressibility, determined in a test A, above 70 N, preferably above 80 N, but excluding compositions prepared by granulating xylitol with a physiologically acceptable, non-cariogenic binder, said binder being present in the range of 0.1 % to 5 % by weight and being selected from the group constituted by polymerized reducing sugars, alkali carboxymethylcellulose and hydrogenated starch hydrolysates.

**2.** Composition according to claim 1, characterized in that the proportion of xylitol is greater than or equal to 60 % by weight and preferably to 80 % by weight.

**3.** Composition according to claim 1 or 2, characterized in that the additive is selected from sugars, glucose syrups, maltodextrins and their hydrogenated derivatives.

**4.** Composition according to claim 3, characterized in that the additive is selected from the group comprising sorbitol, mannitol, maltitol and maltodextrins.

**5.** Process for the fabrication of a directly compressible pulverulent composition, characterized in that a starting material substantially constituted by xylitol and at least one additive selected from saccharides, oligosaccharides and polysaccharides and their corresponding hydrogenated compounds is subjected to an extrusion treatment comprising a thermal treatment zone and at least one extrusion die, the rate of feed of starting material into the apparatus and the parameters of the extrusion treatment, namely the temperature inside the thermal treatment zone, the diameter of the extrusion die and the speed of transport of the starting material inside the thermal treatment zone, being so chosen that the xylitol/additive mixture will be partly molten at the outlet of the die and before its discharge from the latter.

**6.** Process according to claim 5, characterized in that the starting material subjected to the extrusion treatment consists of a quantity of xylitol greater than or equal to 60 % by weight, preferably greater than 80 % by weight.

**7.** Process according to claim 5 or 6, characterized in that the additive is selected from sorbitol, maltitol, mannitol and maltodextrins, the preferred additive being sorbitol.

**8.** Process according to any one of claims 5 to 7, characterized in that the apparatus is of the double screw type comprising at least one extrusion die, the parameters of the extrusion treatment being chosen so that the starting material is at a temperature of from 75 to 110°C inside the die and before discharge of the said material from the latter.

**9.** Process according to any one of claims 5 to 8, in which the pulverulent composition contains an additive consisting of sorbitol, characterized in that the temperature of the starting material inside the die and before its discharge from the latter is preferably from 80 to 105°C.

**10.** Directly compressible pulverulent composition, characterized in that it can be obtained according to the process of any one of claims 5 to 9.

**11.** Use of the pulverulent composition according to any one of claims 1 to 4 and 10 as sweetening filler in tablets or in articles of confectionery, in particular of the chewing-gum type.

**12.** Tablets or articles of confectionery, in particular of the chewing-gum type, characterized in that their sweetening filler consists, at least in part, of the pulverulent composition according to any one of claims 1 to 4 and 10.

**Patentansprüche**

**1.** Direkt verpreßbare pulverförmige Zusammensetzung auf der Basis von Xylitol, dadurch gekennzeichnet, daß sie mindestens einen Zusatzstoff umfaßt, ausgewählt unter den Sacchariden, Oligosacchariden und Polysacchariden sowie den entsprechenden hydrierten Verbindungen, und daß sie eine Komprimierbarkeit, bestimmt in einem Test A, von höher als 70 N, vorzugsweise höher als 80 N aufweist, mit Ausnahme der Zusammensetzungen, die durch Granulation von Xylitol mit einem physiologisch akzeptablen, nicht-kariogenen Bindemittel hergestellt werden, wobei das genannte Bindemittel in einem Verhältnis von 0,1 bis 5 Gew.-% anwesend ist und aus der Gruppe gewählt wird, die aus den polymerisierten reduzierenden Zuckern, den Alkalisalzen von Carboxymethylcellulose und den Hydrolysaten von hydrierter Stärke besteht.

**2.** Zusammensetzung nach Anspruch 1, dadurch gekennzeichnet, daß das Verhältnis von Xylitol höher oder gleich 60 Gew.-% ist und vorzugsweise bei 80 Gew.-% liegt.

**3.** Zusammensetzung nach Anspruch 1 oder 2, dadurch gekennzeichnet, daß der Zusatzstoff unter den Zuckern, den Sirups von Glucose, den Maltodextrinen und ihren hydrierten Derivaten ausgewählt wird.

**4.** Zusammensetzung nach Anspruch 3, dadurch gekennzeichnet, daß der Zusatzstoff aus der Gruppe gewählt wird, die Sorbitol, Mannitol, Maltitol und Maltodextrine umfaßt.

**5.** Verfahren zur Herstellung einer direkt verpreßbaren pulverförmigen Zusammensetzung, dadurch gekennzeichnet, daß man einen Ausgangsstoff, der im wesentlichen aus Xylitol und mindestens einem Zusatzstoff besteht, ausgewählt unter den Sacchariden, Oligosacchariden und Polysacchariden sowie den entsprechenden hydrierten Verbindungen, einer Extrusions-Behandlung unterzieht, die eine Zone der thermischen Behandlung und mindestens eine Extrusions-Düse umfaßt, wobei der Durchsatz der Beschickung der Anlage mit Ausgangsmaterial sowie die Parameter der Extrusions-Behandlung, nämlich die im Inneren der Zone der thermischen Behandlung herrschende Temperatur, der Durchmesser der Extrusions-Düse und die Geschwindigkeit der Bewegung des Ausgangsmaterials im Inneren der Zone der thermischen Behandlung in der Weise gewählt werden, daß am Ausgang der Düse und vor deren Austritt die Mischung Xylitol/Zusatzstoff teilweise geschmolzen ist.

**6.** Verfahren nach Anspruch 5, dadurch gekennzeichnet, daß der der Extrusion unterzogene Ausgangsstoff eine Menge an Xylitol von höher oder gleich 60 Gew.-% aufweist, vorzugsweise von höher oder gleich 80 Gew.-%.

**7.** Verfahren nach Anspruch 5 oder 6, dadurch gekennzeichnet, daß der Zusatzstoff unter Sorbitol, Mannitol, Maltitol und den Maltodextrinen ausgewählt wird, wobei der bevorzugte Zusatzstoff Sorbitol ist.

**8.** Verfahren nach einem der Ansprüche 5 bis 7, dadurch gekennzeichnet, daß die Anlage ein Zweischnecken-Typ ist und mindestens eine Extrusions-Düse umfaßt, wobei die Parameter der Extrusions-Behandlung in der Weise gewählt werden, daß der Ausgangsstoff im Inneren der Düse und vor seinem Austritt aus dieser eine Temperatur zwischen 75 °C und 110 °C besitzt.

**9.** Verfahren nach einem der Ansprüche 5 bis 8, bei dem die pulverförmige Zusammensetzung einen aus Sorbitol bestehenden Zusatzstoff umfaßt, dadurch gekennzeichnet, daß die Temperatur des Ausgangsstoffes im Inneren der Düse und vor seinem Austritt aus dieser vorzugsweise zwischen 80 °C und 105 °C liegt.

10. Direkt verpreßbare pulverförmige Zusammensetzung, dadurch gekennzeichnet, daß sie geeignet ist, durch das Verfahren nach einem der Ansprüche 5 bis 9 erhalten zu werden.

11. Verwendung der pulverförmigen Zusammensetzung nach einem der Ansprüche 1 bis 4 und 10 als Süßungsmittel-Zusatz in Tabletten oder in Süßwaren, insbesondere vom Typ Kaugummi.

12. Tabletten oder Süßwaren, insbesondere vom Typ Kaugummi, dadurch gekennzeichnet, daß ihr Süßungsmittel-Zusatz mindestens teilweise aus der pulverförmigen Zusammensetzung nach einem der Ansprüche 1 bis 4 und 10 besteht.

# FIG.1.

FIG.2.